# EUROPEAN PATENT APPLICATION

(11) **EP 0 812 816 A1**
(43) Date of publication of application: **17.12.1997**
(21) Application number: 96900697.2
(22) Date of filing: 17.01.1996
(51) Int. Cl.: C07C 50/02, C07C 50/10, C07C 46/06, B01J 23/40, B01J 23/62, B01J 23/64

(54) **PROCESS FOR PRODUCING QUINONES**

(30) Priority: 20.01.1995 JP 7375/95; 20.01.1995 JP 7419/95
(71) Applicant: Seiko Kagaku Kabushiki Kaisha, Tokyo 101 (JP)
(72) Inventor: YOSHIZAWA, Toshio Seiko Kagaku Kabushiki Kaisha,, Saitama-ken 333 (JP); ITO, Masayuki Seiko Kagaku Kabushiki Kaisha, 15-33, Saitama-ken 333 (JP); FURUYAMA, Yuka Seiko Kagaku Kabushiki Kaisha 15-33, Saitama-ken 333 (JP); TAKAOKA, Masatoshi Seiko Kagaku Kabushiki Kaisha,, Saitama-ken 333 (JP); KANO, Harukichi Seiko Kagaku Kabushiki Kaisha,, Saitama-ken 333 (JP); ICHIKAWA, Yataro Seiko Kagaku Kabushiki Kaisha,, Saitama-ken 333 (JP)
(74) Representative: LOUIS, PÖHLAU, LOHRENTZ & SEGETH
(86) International application number: JP9600052
(87) International publication number: WO9622269

(57) **Abstract**

Provided is a process for preparing quinone in high purity, in high quality and in high yield in industrially easy manner and with no specific equipment and machinery and with no specific operations. In liquid phase, an aromatic diol compound is reacted with peroxide in the presence of a catalyst with a component I containing one or more elements selected from the group consisting of platinum, palladium, rhodium, ruthenium, osmium, iridium and rhenium.

## Description

### TECHNICAL FIELD

The present invention relates to a process for preparing quinone in high purity, in high quality and in high yield by reacting an aromatic diol compound with an oxidizing agent, particularly peroxide.

### BACKGROUND ART

Quinones are much useful compounds as polymerization inhibitors, stabilizers for unsaturated polyesters, starting materials for aromatic polyesters and intermediates for pharmaceuticals, agricultural chemicals and dye stuffs.

Conventionally, many processes are known for preparation of quinones through oxidization of aromatic diol compounds. Typical ones among them are exemplified below.

For example, JP-A-91-287,557 discloses a process for oxidizing hydroquinone with sodium chlorate in the presence of 2% aqueous sulfuric acid and vanadium pentoxide in an inactive organic solvent.

French Patent No. 1,338,462 discloses a process for oxidizing hydroquinone with oxygen in the presence of 5% supported ruthenium or rhodium catalyst in glacial acetic acid as a solvent.

Moreover, Ind. End. Chem. Prod. Res. Dev., Vol. 21, 566 (1982) discloses a process for oxidizing hydroquinone with oxygen in the presence of supported ruthenium or rhodium catalyst in a mixed solvent of 2-propanol, acetonitrile and acetic acid or 2-propanol and acetonitrile.

US patent No. 5,118,823 discloses a process for oxidizing hydroquinone with oxygen in the presence of 5% platinum/carbon in acetic acid as a solvent.

JP-B-93-79,054 discloses a process for reacting hydroquinone with hydrogen peroxide in the presence of an iodine compound in a mixture of water, acidic aqueous solution and inactive polar organic solvent or in a mixture of water and inactive polar organic solvent.

Among the above-mentioned prior art, the processes disclosed in JP-A-91-287,557 and JP-B-93-79,054, which can obtain intended products in high yield, have inevitable inclusion of halides or halogen ions due to secondarily produced NaCl and/or the iodine compound used as the catalyst, resulting in qualitative problems. Moreover, the catalytic iodine compound is difficult to recover.

The processes disclosed in French Patent No. 1,338,462, Ind. End. Chem. Prod. Res. Dev., Vol. 21, 566 (1982) and US Patent No. 5,118,823 have reactions carried out in limited solvents such as acetic acid and glacial acetic acid and have difficulties in isolation of intended products. The processes have, furthermore, many economical and operative problems such that a great volume of precious metal catalyst must be employed and that the reactions have to be carried out under increased pressure.

After all, carrying out any of the prior art in industrial scale would involve various bars to be overcome, such as use of costly catalysts in great volume, difficulties in recovery, further improvements desired in quantity of intended products and uneasiness in isolation of the intended products.

It is an object of the present invention to provide a process for preparing quinone in highly purity and in high yield through industrially simple operations.

### DISCLOSURE OF THE INVENTION

The inventors have made intensive studies and researches to overcome the above-mentioned problems in the prior art, thus completing a process for preparing quinone in an industrially much advantageous manner.

More specifically, the present invention is characterized in that, in liquid phase, an aromatic diol compound is reacted with peroxide in the presence of a catalyst which comprises a components I containing one or more elements selected from the group consisting of platinum, palladium, rhodium, ruthenium, osmium, iridium and rhenium and is further characterized in that, in liquid phase, an aromatic diol compound is reacted with an oxidizing agent in the presence of a catalyst which comprises a component I containing one or more elements selected from the group consisting of platinum, palladium, rhodium, ruthenium, osmium, iridium and rhenium and in the presence of a catalyst which comprises a component II containing one or more elements selected from the group consisting of bismuth, lead, thallium, indium, tin, arsenic, selenium, antimony and tellurium.

A process for producing quinone according to the present invention has simple production steps and good operativity. Quinones are produced in high yield, in good appearance and in high stability.

In the present invention, an aromatic diol compound is reacted with an oxidizing agent in a solvent in the presence of a catalyst. This is represented by a following reaction formula (I):

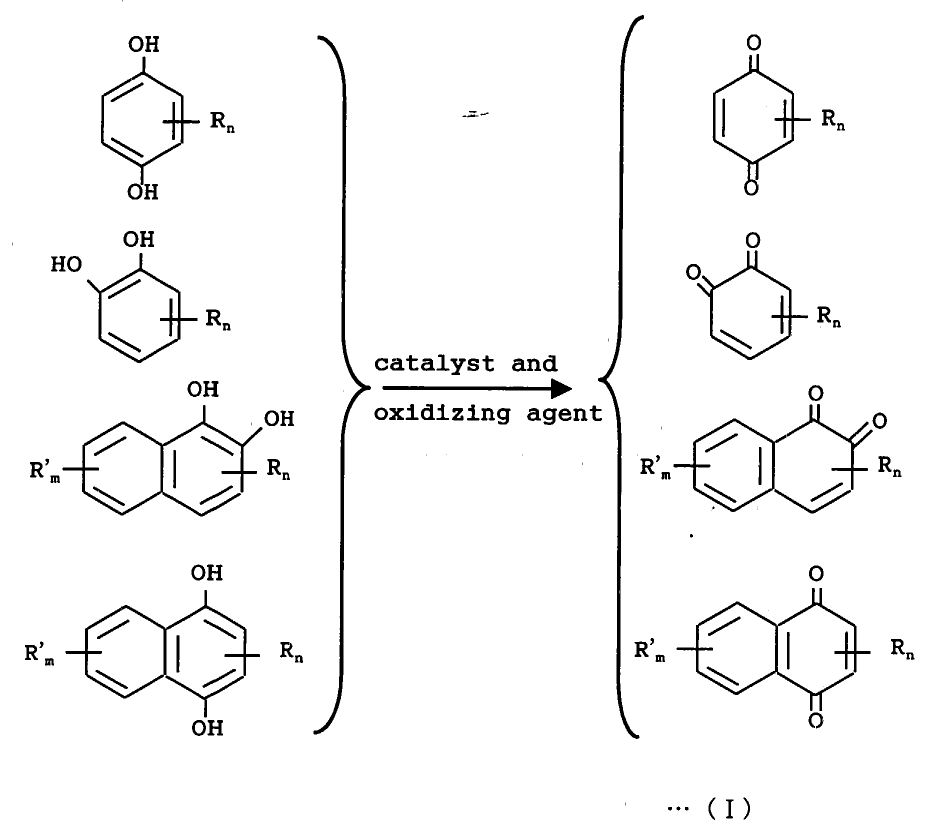

Any aromatic diol compound may be used in the present invention providing that it is directly coupled to aromatic ring and has two or more hydroxyl groups and that it may be converted into quinone according to the process of the present invention. For example, it may be a compound represented by the following formula (II), (III), (IV) or (V): 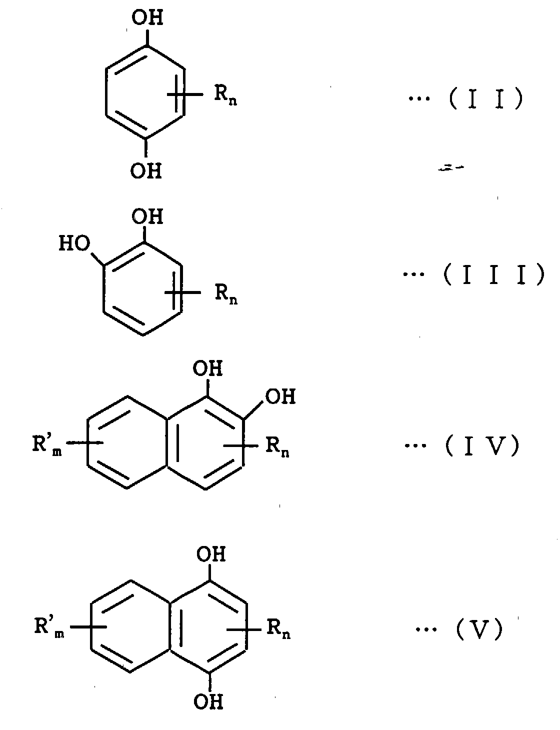 wherein R and R' respectively represent hydrogen atom or straight- or branched-chain alkyl, cyclohexyl, phenyl or halogen having 1 to 8 carbon atoms, and n and m are numbers of 1-4. More specifically, it may be, for example, hydroquinone, catechol, methylhydroquinone, t-butylhydroquinone, 2,5-di-t-butylhydroquinone, octylhydroquinone, 2,5-dioctylhydroquinone, cyclohexyl-1,4-benzenediol, phenyl-1,4-benzenediol, 2,5-diphenyl-1,4-benzendiol, 1,2-naphthalediol, 1,4-naphthalenediol, 2-methyl-1,4-naphthalenediol or chlorohydroquinone.

Any solvent may be used in the present invention, providing that it does not adversely affect the reaction. Such solvent may be, for example, selected from the group consisting of water, alcohol, aromatic hydrocarbon, ketone, ether, ester, glycol or a mixture of two or more thereof. Preferably, the compound may be for example, water, methanol, ethanol, isopropyl alcohol, ethylene glycol, toluene, xylene, acetone or a mixture thereof. Most preferably, the compound may be, for example, methanol, a mixture of methanol with water, acetone, a mixture of acetone with water or a mixture of toluene with water.

There is no particular limitation on an amount of the solvent employed. However, operatively preferred is an amount of solvent with which the generated quinone is soluble. Generally, such amount is 0.5-50 times, preferably 0.5-20 times, most preferably 1-10 times by weight as much as that of the starting materials.

A catalyst used in the present invention may have a component I having one or more elements selected from the group consisting of platinum, palladium, rhodium, ruthenium, osmium, iridium and rhenium. Furthermore, it may have mixed components of the above-mentioned component I with a component II having one or more elements selected from the group consisting of bismuth, lead, thallium, indium, tin, arsenic, selenium, antimony and tellurium. The catalysts which comprise the above-mentioned component I or the mixed components of the component I with the component II may be employed as single metal or metal compound or in the form supported by a carrier. The carrier used may be activated carbon, silica, alumina, silica-alumina, zeolite or a mixture thereof. Preferably, the catalyst used may be a supported catalyst in the form of one or more elements selected from the group consisting of platinum, rhodium and ruthenium and supported by activated carbon or alumina.

There is no specific limitation on an amount of the catalyst used. However, a preferable amount may differ depending upon the kind of an oxidizing agent used. In the case of using hydrogen peroxide, industrially the catalytic amount will suffice as the amount of the catalyst used. Generally, the amount may be 0.1-10%, especially preferably 0.5-5% by weight of that of the aromatic diol compound. The catalyst may be repeatedly used without lowering of its activity.

The oxidizing agent of the present invention may be peroxide such as hydrogen peroxide, alkylhydroperoxide, percarboxylic acid, peracid ester or dialkyl peroxide, oxygen, air or oxygen-containing mixed gas. It may be preferably hydrogen peroxide, alkylhydroperoxide, percarboxylic acid, molecular oxygen and air; and it may be most preferably hydrogen peroxide.

In the case of using hydrogen peroxide, it is generally in the form of aqueous solution. There is no specific limitation on its concentration. Industrially readily available 35 or 60% aqueous hydrogen peroxide may be employed as it is or in the form of suitable dilution. The amount used is preferably 0.8-2.0 times, most preferably 1.0-1.2 times by mole weight to that of the aromatic diol compound.

In the case of employing molecular oxygen or air as the oxidizing agent, the amount used may be 5-200%, preferably 10-100% by weight to that of the aromatic diol compound. In this case, a large amount of the catalyst is needed; but this causes no industrial disadvantage since the catalyst may be reused without lowering of its activity. In the case of using molecular oxygen or air, the reaction may be carried out either under atmospheric pressure or under elevated pressure.

Any reaction temperature may be used, providing that the process of the present invention may be carried out. It is generally 0-100°C, preferably 10-80°C, and most preferably 20-50°C. In an example of operation for ordinary reaction, an aromatic diol compound, a solvent and a catalyst are placed in room temperature and aqueous hydrogen peroxide is added dropwise. After completion of the dropwise addition, the reaction temperature is increased to 40-50°C which temperature is maintained for predetermined hours, thus completing the reaction.

In the case of using a solid catalyst, the catalyst may be readily recovered for reuse by filtering the reaction product which has been obtained after completion of the reaction in the above-mentioned manner. A filtrate may be used for next usage as it is or may be concentrated and cooled to precipitate quinone as crystals. The quinone precipitated may be readily obtained as crystals by filtration. The quinone thus obtained is of high quality and high purity and may be used without refining. The solvent used for the reaction may be recovered for reuse by simple distillation. That which includes water may be also reused for the reaction as it is.

### BEST MODE FOR CARRYING OUT THE INVENTION

### [Examples]

The present invention is further disclosed with reference the following examples. It is to be, however, noted that the present invention is not limited to the examples.

### EXAMPLE 1

55g (0.5 mole) of hydroquinone, 500ml of methanol and 1g of 5% ruthenium/carbon were placed in a four-necked round-bottomed flask with a capacity of 1 liter and equipped with an agitator, a dropping funnel and a thermometer. After starting agitation, 53.4g (0.55 mole as hydrogen peroxide) of 35% aqueous hydrogen peroxide was added dropwise over about 3 hours while a reaction temperature is maintained to 25-30°C. After completion of the dropwise addition, the reaction mixture was maintained for about 3 hours at 25-30°C for maturation, thus completing the reaction. After completion of the reaction, the reaction liquid was suction-filtered to recover the catalyst. The filtrate was analyzed with high performance liquid chromatography (HPLC) for quantitative analysis of the reaction product.

As a result of the analysis, no unreacted hydroquinone was detected and the reaction product comprises only p-benzoquinone with yield of 99.9% (with respect to the placed hydroquinone; this is similarly applied hereinafter).

### EXAMPLE 2

The reaction was made under the same conditions in the procedure of EXAMPLE 1 except that 5% rhodium/carbon was used as the catalyst and the maturation temperature was 40-45°C. No unreacted hydroquinone was detected and the yield of p-benzoquinone was 99.8%.

### EXAMPLE 3

The reaction was made under the same conditions as those in the procedure of EXAMPLE 1 except that 5% platinum/carbon was used as the catalyst and that the maturation was made at 40-45°C for 2 hours. Unreacted hydroquinone was present at 3% and the yield of p-benzoquinone was 96%.

### EXAMPLE 4

The reaction was made under the same conditions as those in the procedure of EXAMPLE 1 except that 5% platinum/carbon was used as the catalyst, that 63.1g (0.65 mole) of 35% aqueous hydrogen peroxide was used and that the maturation temperature was 40-45°C. Unreacted hydroquinone was present at 1% and the yield of p-benzoquinone was 98.5%.

### EXAMPLE 5

The reaction was made under the same conditions as those in the procedure of EXAMPLE 1 except that 2g of 5% platinum/carbon was used as the catalyst. No unreacted hydroquinone was detected and the yield of p-benzoquinone was 99.3%.

### EXAMPLE 6

The reaction was made under the same conditions as those in the procedure of EXAMPLE 1 except that 5% palladium/carbon was used as the catalyst and the maturation temperature was 40-45°C. Unreacted hydroquinone was present at 5% and the yield of p-benzoquinone was 94.5%.

### EXAMPLE 7

The reaction was made under the same conditions as those in the procedure of EXAMPLE 1 except that 5% ruthenium/alumina was used as the catalyst. No unreacted hydroquinone was detected and the yield of p-benzoquinone was 99.6%.

### EXAMPLE 8

The reaction was made under the same conditions as those in the procedure of EXAMPLE 1 except that 5% platinum and 5% ruthenium/carbon was used as the catalyst. No unreacted hydroquinone was detected and the yield of p-benzoquinone was 99.7%.

### EXAMPLE 9

The reaction was made under the same conditions as those in the procedure of EXAMPLE 1 except that 5% iridium/carbon was used as the catalyst and that 200 ml of acetone was used as the solvent. No unreacted hydroquinone was detected and the yield of p-benzoquinone was 99.9%.

### EXAMPLE 10

The reaction was made under the same conditions as those in the procedure of EXAMPLE 1 except that 200 ml of acetone was used as the solvent. No unreacted hydroquinone was detected and the yield of p-benzoquinone was 99.9%.

### EXAMPLE 11

The reaction liquid in EXAMPLE 10 was filtered to recover the 5% ruthenium/carbon. The filtrate was cooled to 10°C to precipitate p-benzoquinone as crystals which were then filtered and dried. As a result, 35.1g of p-benzoquinone was obtained whose purity was 99.9% both in HPLC and in titration.

### EXAMPLE 12

The reaction was made under the same conditions as those in the procedure of EXAMPLE 1 except that the recovered 5% ruthenium/carbon and the filtered mother liquid in EXAMPLE 11 were reused. No unreacted hydroquinone was detected and the yield of p-benzoquinone was 99.9%. The reaction liquid was after-treated in the manner shown in EXAMPLE 11 to obtain 53.5g of p-benzoquinone (yield: 99.1%). Its purity was 99.9%. The color appearance was 5Y 8-14 (by standard color cards based on JIS Z 8721). Its purity and color appearance were re-detected after a lapse of 8 months to find out that no changes were seen. Thus, confirmed was its excellent stability in storage.

### EXAMPLE 13

The reaction was made under the same conditions as those in the procedure of EXAMPLE 1 except that the aromatic diol compound was methylhydroquinone. No unreacted methylhydroquinone was detected and the yield of 2-methyl-p-benzoquinone was 99.8%.

### EXAMPLE 14

The reaction was made under the same conditions as those in the procedure of EXAMPLE 1 except that the aromatic diol compound was 62g (0.5 mole) of methylhydroquinone and 250 ml of acetone was used as the solvent. No unreacted methylhydroquinone was detected and the yield of 2-methyl-p-benzoquinone was 99.1%.

### EXAMPLE 15

The reaction was made under the same conditions as those in the procedure of EXAMPLE 1 except that the aromatic diol compound was 83.1g (0.5 mole) of t-butylhydroquinone and 250 ml of acetone was used as the solvent. No unreacted t-butylhydroquinone was detected and the yield of t-butyl-p-benzoquinone was 99.9%.

### EXAMPLE 16

The reaction was made under the same conditions as those in the procedure of EXAMPLE 1 except that the aromatic diol compound was 111.2g (0.5 mole) of 2,5-di-t-butylhydroquinone and 500 ml of acetone was used as the solvent. No unreacted 2,5-di-t-butylhydroquinone was detected and the yield of 2,5-di-t-butylbenzoquinone was 98.5%.

### EXAMPLE 17

The reaction was made under the same conditions as those in the procedure of EXAMPLE 1 except that the aromatic diol compound was 80g (0.5 mole) of 1,4-naphthalenediol and 500 ml of acetone was used as the solvent. Ureacted 1,4-naphthalenediol was present at 2.7% and the yield of 1,4-naphthoquinone was 97.0%.

### EXAMPLE 18

55g (0.5 mole) of hydroquinone, 500 ml of methanol and 1g of supported catalyst in the form of platinum and bismuth supported by activated carbon (5% platinum and 1% bismuth/carbon; such expressions are used hereinafter) were placed in a four-necked round-bottomed flask with the capacity of 1 liter and equipped with an agitator, a dropping funnel and a thermometer. After starting agitation, 53.4g (0.55 mole as hydrogen peroxide) of 35% aqueous hydrogen peroxide was added dropwise over about 3 hours while maintaining a reaction temperature of 25-30°C. After completion of the dropwise addition, the reaction mixture was maintained at 25-30°C for about 3 hours for maturation, thus completing the reaction. After completion of the reaction, the reaction liquid was suction-filtered to recover the catalyst. A filtrate was analyzed with HPLC for quantitative analysis of the reaction product.

As a result of the analysis, no unreacted hydroqinone was detected and the yield of p-benzoquinone was 99.8%.

### EXAMPLE 19

The reaction was made under the same conditions as those in the procedure of EXAMPLE 18 except that the amount of the catalyst and the maturation temperature were 0.5g and 40-45°C, respectively. No unreacted hydroquinone was detected and the yield of p-benzoquinone was 99.9%.

### EXAMPLE 20

The reaction was made under the same conditions as those in the procedure of EXAMPLE 18 except that 1g of 5% platinum/carbon and 0.022g of bismuth nitrate 5 hydrate were used as the catalysts and the maturation temperature was 40-45°C for 2 hours. Unreaced hydroquinone was present at 1.0% and the yield of p-benzoquinone was 98.7%.

### EXAMPLE 21

The reaction was made under the same conditions as those in the procedure of EXAMPLE 18 except that 1g of 5% palladium/carbon and 0.022g of bismuth nitrate 5 hydrate were used as the catalysts and the maturation temperature was 40-45°C. No unreacted hydroquinone was detected and the yield of p-benzoquinone was 99.5%.

### EXAMPLE 22

The reaction was made under the same conditions as those in the procedure of EXAMPLE 18 except that 1g of 5% platinum/carbon and 0.01g of thallium nitrate 3 hydrate were used as the catalysts. Unreacted hydroquinone was present at 2.0% and the yield of p-benzoquinone was 97.8%.

### EXAMPLE 23

The reaction was made under the same conditions as those in the procedure of EXAMPLE 21 except that 0.2g of 5% platinum and 9% lead/carbon was used as the catalyst for 11g of hydroquinone and 10.7g of 35% aqueous hydrogen peroxide was added dropwise for 1 hour. Unreacted hydroquinone was present at 6.0% and the yield of p-benzoquinone was 94.0%.

### EXAMPLE 24

The reaction was made under the same conditions as those in the procedure of EXAMPLE 18 except that the aromatic diol compound was 1,4-naphthalenediol. No unreacted 1,4-naphthalenediol was detected and the yield of 1,4-naphthoquinone was 96.5%.

### EXAMPLE 25

The reaction was made under the same conditions as those in the procedure of EXAMPLE 18 except that the aromatic diol compound was methylhydroquinone. No unreaced methylhydroquinone was detected and the yield of 2-methyl-p-benzoquinone was 99.9%.

### EXAMPLE 26

4.4g (0.04 mole) of hydroquinone, 100 ml of methanol and 5.5g of 5% platinum and 1% bismuth/carbon were placed in a four-necked flask with the capacity of 300 ml and equipped with an agitator, a thermometer and a gas inlet. After starting agitation, air was introduced at 100 ml/min at room temperature. The reaction was stopped after a lapse of 6 hours and the reaction liquid was analyzed. Unreacted hydroquinone was present at 2% and the yield of p-benzoquinone was 98%.

### EXAMPLE 27

The reacton was made under the same conditions as those in the procedure of EXAMPLE 26 except that the quantity of hydroquinone was 5.5g (0.05 mole) and 100 ml of acetone was used as the solvent Unreacted hydroquinone was present at 5% and the yield of p-benzoquinone was 95%.

### EXAMPLE 28

The reaction was made under the same conditions as those in the procedure of Example 18 and the reaction liquid was filtered to recover the catalyst. The filtrate was cooled to 20°C to precipitate p-benzoquinone as crystals which were then filtered and dried. Thus, 22g of p-benzoquinone was obtained whose purity was 99.9% both in HPLC and in titration.

### EXAMPLE 29

The reaction was made under the same conditions as those in the procedure of EXAMPLE 18 except that the recovered catalyst and the filtered mother liquid in EXAMPLE 28 were reused. The reaction liquid was after-treated in the same manner as that in EXAMPLE 28 to obtain 53.8g of p-benzoquinone (yield: 99.6%). Its purity was 99.9% and its color appearance was 5Y 8-14 (by standard color cards based on JIS Z 8721). Its purity and color appearance were redetected after a lapse of 8 months to find out that no changes were seen. Thus, confirmed was its excellent stability in storage.

### CAPABILITY OF EXPLOITATION IN INDUSTRY

According to the present invention, quinones can be prepared in high purity, in high quality and in high yield in industrially easy operations and with no specific equipment and machinery and with no specific operations. The process is much economical and has no specific problems in safety and sanitation and in environmental pollution.

## Claims

1. A process for preparing quinone characterized in that, in liquid phase, an aromatic diol compound is reacted with peroxide in the presence of a catalyst which comprises a component I containing one or more elements selected from the group consisting of platinum, palladium, rhodium, ruthenium, osmium, iridium and rhenium.

2. A process for preparing quinone characterized in that, in liquid phase, an aromatic diol compound is reacted with an oxidizing agent in the presence of a catalyst which comprises a component I containing one or more elements selected from the group consisting of platinum, palladium, rhodium, ruthenium, osmium, iridium and rhenium and in the presence of a catalyst which comprises a component II containing one or more elements selected from the group consisting of bismuth, lead, thallium, indium, tin, arsenic, selenium, antimony and tellurium.

3. A process according to claim 2 wherein the oxidizing agent is peroxide.

4. A process according to claim 1 wherein the catalyst with the component I contains one or more elements selected from the group consisting of platinum, rhodium and ruthenium.

5. A process according to claim 2 wherein the catalyst with the component I contains one or more elements selected from the group consisting of platinum, rhodium and ruthenium and the catalyst with the component II contains one or more elements selected from the group consisting of bismuth and lead.

6. A process according to claim 1 or 4 wherein the catalyst with the component I is a supported catalyst which is supported by activated carbon, silica, alumina, silica-alumina or zeolite.

7. A process according to claim 2 or 5 wherein the catalysts with the components I and II are supported catalysts which are supported by activated carbon, silica, alumina, silica-alumina or zeolite.

8. A process according to claim 1 or 4 wherein the peroxide is hydrogen peroxide, organic peracid or alkylhydroperoxide.

9. A process according to claim 2 or 5 wherein the oxidizing agent is hydrogen peroxide, organic peracid, alkylhydroperoxide, oxygen, air or an oxygen-containing mixed gas.

10. A process according to claim 2 or 5 wherein the oxidizing agent is hydrogen peroxide.

11. A process according to claim 1 or 2 wherein the aromatic diol compound is hydroquinone, catechol, methylhydroquinone, t-butylhydroquinone, 2,5-di-t-butylhydroquinone, octylhydroquinone, 2,5-dioctylhydroquinone, cyclohexyl-1,4-benzenediol, phenyl-1,4-benzenediol, 2,5-diphenyl-1,4-benzenediol, 1,2-naphthalenediol, 1,4-naphthalenediol, 2-methyl-1,4-naphthalenediol or chlorohydroquinone.

12. A process according to claim 1 or 2 wherein a solvent is water, alcohol, aromatic hydrocarbon, ketone, ether, ester, glycol or a mixture of two or more thereof.

13. A process according to claim 1 or 2 wherein a solvent is water, methanol, ethanol, isopropyl alcohol, ethylene glycol, toluene, xylene, acetone, methyl ethyl ketone, methyl isobutyl ketone or a mixture of two or more thereof.
